(19) Europäisches Patentamt / European Patent Office / Office européen des brevets

(11) **EP 4 389 003 B1**

(12) **EUROPEAN PATENT SPECIFICATION**

(45) Date of publication and mention of the grant of the patent:
**13.08.2025  Bulletin 2025/33**

(21) Application number: **22215428.8**

(22) Date of filing: **21.12.2022**

(51) International Patent Classification (IPC):
*A61B 5/339* (2021.01)    *A61B 5/346* (2021.01)
*A61B 5/367* (2021.01)    *A61B 5/00* (2006.01)
*A61B 5/361* (2021.01)

(52) Cooperative Patent Classification (CPC):
A61B 5/339; A61B 5/346; A61B 5/361;
A61B 5/367; A61B 5/6852; A61B 5/6869;
A61B 5/743

(54) **METHOD FOR ANALYSING AN INTRACARDIAC ELECTROGRAM**

VERFAHREN ZUR ANALYSE EINES INTRAKARDIALEN ELEKTROGRAMMS

PROCÉDÉ D'ANALYSE D'UN ÉLECTROGRAMME ENDOCAVITAIRE

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR

(43) Date of publication of application:
**26.06.2024  Bulletin 2024/26**

(73) Proprietor: **CathVision ApS**
**2200 Copenhagen (DK)**

(72) Inventors:
• **PAAMAND, Rune**
**3050 Humlebaek (DK)**
• **PIETRZAK, Grzegorz**
**02-787 Warsaw (PL)**
• **LAWS, Michael**
**Quarndon, Derbyshire DE22 5JS (GB)**

(74) Representative: **Gottschald**
**Patentanwälte Partnerschaft mbB**
**Klaus-Bungert-Straße 1**
**40468 Düsseldorf (DE)**

(56) References cited:
US-A1- 2007 232 949    US-A1- 2018 368 713
US-A1- 2020 245 885    US-A1- 2022 344 025

**Description**

The present invention relates to a method for analysing an intracardiac electrogram according to claim 1, to a control system configured to perform the proposed method according to claim 14.

The present method is particularly concerned with atrial fibrillation and atrial flutter. Electrically, atrial fibrillation is chaotic activation of muscle cells of the atria. During atrial fibrillation, the atria only minimally contribute to the function of the heart. Atrial fibrillation, therefore, reduces the output of the heart but is not immi-nently dangerous. However, when becoming chronic, atrial fibrillation is correlated with increased morbidity and mortality. One treatment option for atrial fibrillation is ablation therapy. Ablation is the destruction of the cells that allow electrical wave re-entry to reduce chaotic activation of the atrial muscle cells.

An ablation therapy with a comparably high success rate regarding restoration and medium term maintenance of sinus rhythm is stepwise ablation. For stepwise ablation multiple ablation techniques and ablation targets are successively performed. Stepwise ablation may be ended without performing all possible ablation steps at the discretion of the physician. Stepwise ablation is usually ended when the patient reverts to sinus rhythm. However, such procedures may be long and a large fraction of patients require a second ablation procedure.

The result of stepwise ablation and other ablation therapies depends lot on the experience of the physician. Many different ways of processing and presenting measured data of intracardiac electrograms prior to, during and after ablation therapy exist. For example, prior to ablation therapy sophisticated 3D-mapping systems can be used. Those mapping systems create an anatomical map of the target anatomical structure, of the left atria, and show multiple features on the 3D-anatomical map. Other mapping systems exist, that display a derived feature with a time dimension, e.g. US 2018/0368713 A1 and US 2007/0232949 A1.

Other known control systems usable during ablation therapy like GE Prucka 3 overlay multiple features derived from the intracardiac electrogram on a small-time window of the intracardiac electrogram.

With the known data processing control systems discovering the effect of an ablation therapy and prognosing the effect of the ablation therapy on the patient remains a challenge. By providing a physician with a better representation of the physiological processes happening in reaction to the ablation therapies presented in form of features derived from intracardiac electrograms the success of ablation therapies can be increased.

It is a challenge to improve on the known prior art to increase the success of ablation therapies by providing a physician and/or an automated decision system.

The invention is based on the problem of improving the known methods and control systems such that a further optimization regarding the named challenge is reached.

The above-noted object is solved by the features of claim 1.

The main realization of the present invention is that conventional control systems rely on deriving and displaying features with very high time resolution to give the physician a good overview of the current status of the heart of the patient at any given time. However, it is hard to interpret absolute values of features relating to human physiology in general as the inter- and intra-person variability of absolute values can be higher than the actual signal variability. It has been realized that a more holistic approach focused on deriving and processing information with a focus on the general context and not on the absolute values leads to a better understanding of the changes in the heart caused by the ablation therapy.

The idea behind the proposed teaching is to aggregate multiple derived features of multiple locations into a single feature space to allow the analysis of local and global changes of the interrelation of the different physiological processes happening in the heart. This aggregated approach allows detecting changes in the functioning of the heart caused by the ablation therapy by comparing data previous to ablation events with data after the ablation events on a common intensity range. It is proposed to transform different features which may well have different units onto a single intensity range that shows changes of those features over a broader timescale. By having multiple features with a single intensity range it becomes possible to see, analyse and differentiate local and global changes that cannot be expressed in a single feature but instead show themselves in a concurrent change of multiple features. Those changes become visible by analysing the changes of multiple features over a certain time irrespective of the absolute values of those features. To sum it up, the proposed solution allows seeing actual reactions to ablation therapy which can then be used to decide on further steps of the ablation therapy.

Proposed is a method for analysing an intracardiac electrogram via a control system, wherein the intracardiac electrogram has been recorded via a catheter inserted into a human body and has a time length, wherein the catheter comprises multiple electrodes placed at different anatomical positions, wherein multiple channels of the intracardiac electrogram have been recorded by the electrodes, wherein the control system groups the channels into at least a first group of channels comprising at least one channel assigned to a first anatomical region and a second group of channels comprising at least one channel assigned to a second anatomical region, wherein the control system derives derived features comprising a first derived feature relating to the first anatomical region and a different second derived feature relating to the first anatomical region from the first group of channels and derives the first derived feature and the second derived feature relating to a second anatomical region from the second group of channels, wherein the control system derives data points of the derived features in intervals over the time length, the first and second features thereby each having a time dimension and a value dimension with a value range, wherein the control system combines the derived features into a feature space comprising at least an anatomical dimension and a feature dimension and a time dimension and an intensity dimension, wherein the anatomical dimension comprises the anatomical regions, wherein the feature dimension comprises the derived features, wherein the time dimension is the time dimension of the first and the second derived features, wherein the intensity dimension comprises an intensity range and wherein the control system transforms the value ranges of the derived features onto the intensity range.

The proposed solution has two main application focuses. The first focus relates to the visual representation of the feature space for use by a physician. The second focus is the use of the feature space in an automated decision system.

Preferred embodiments relate to displaying on a display of the control system the feature space. Very preferred embodiments of claim 2 relate to the aggregation of data along the time dimension and to displaying the derived features as rows along a common time axis. The intensity range is preferably displayed as a heatmap. The display of several minutes of aggregated data allows a physician to gain insight into the changes happening over several features over a timescale of minutes and thereby gaining insides about a substantial part of the ablation therapy.

For further visual enhancement in one embodiment according to claim 3 the control system low pass filters intensity values of the derived features along the time dimension. This further underlines the idea of the present invention to show significant changes over a broad timescale and prioritize these over fine granular absolute values.

Another preferred embodiment according to claim 3 relates to visually grouping the derived features into visual groups such that a group comprises the anatomical regions for each feature. In this way it becomes easily manageable to compare changes in a feature between anatomical regions. For example, if one anatomical region relates to the left atrium and another anatomical region relates to the right atrium, it becomes possible to see at one glance if a feature has changed globally or only for one atrium that is currently ablated.

Claim 4 relates to the possibility of showing at least one channel of a surface ECG or the intracardiac electrogram in addition to the feature space. The comparison between the aggregated feature space showing a broad time window and a channel which is well known to physicians allows detecting a significant change in the display of the feature space and then reverting to the analysis of a short time excerpt of the channel in the way that the physician is used to. The channel provides more detail for the current time.

Claim 5 relates to the preferred aggregation over time by deriving the features. It shows how the proposed method values overview over preciseness.

Another interesting embodiment is subject of claim 6. It relates to inserting a surface feature derived from the surface ECG into the feature space. This embodiment shows the flexibility of the proposed approach as even quite different features may be combined for a global overview of what is happening in the heart during the ablation therapy.

In a preferred embodiment according to claim 7 the control system may constantly receive new data from a measurement system and update the feature space and preferably the display in real-time.

The invention also relates to the transformation functionality by the control system to transform the value ranges of the derived features onto the intensity range. The transformation functions differ between features. This is obviously the case if the features have different units and therefore fundamentally different values. To enhance inter-feature comparability, according to claim 8 the transformation function per feature may be the same for all anatomical groups.

Further, the transformation functions may be linear or non-linear. Non-linear transformation functions have the advantage of being adaptable to expected and/or occurring changes while at the same time reducing the influence of physiological variability. Non-linear transformation functions are well-known, but not used in the current field because they make the relation of the transformed values non-obvious and unintuitive to the user. Here, that is used to gain a better insight into changes at the cost of knowledge about absolute values.

Another interesting feature described in claim 8 relates to the possibility of updating the transformation functions based on the value ranges of a single feature or of all features that are the same feature derived for different anatomical regions. Another possibility of designing the transformation functions is setting boundaries, in particular based on physiological knowledge. For example, the midpoint of a linear transformation might be forced into a certain range of physiological values. Claim 9 also relates to the possibility of updating the transformation functions with values of data-points of the respective feature that are located "in the future".

Changing already calculated values for a feature at a certain time retrospectively or by including future values in the calculation is rather unusual but fits very well to the proposed solution. It is another way of sacrificing accuracy to improve the visual overview of changes in the behavior of the heart. Changes in the electrical activation of the heart cannot always be predicted. Retrospectively redefining the transformation functions allows the physician to view older data in light of the changes that happened more recent.

According to claim 10 there may be at least three groups of channels and/or at least two channels per a group. Generally, the selection of the number of groups and/or the number of channels per group can be adapted to the actual ablation procedure and the used catheter. Choosing three groups is adapted to a number of usual ablation proceedings and may in particular allow having one group for the right atrium and one group for the left atrium and possibly another group in between. Grouping multiple channels into one group, in particular if the channels in a group relate to the same anatomical structure, increases the robustness of the derived features. At the same time, not including channels into a group that are of a different anatomical structure improves the precision of the feature. For example, the left atrium and the right atrium may behave very different during the ablation of one of them.

Preferred features of the feature dimension are given in claim 11. These have been carefully chosen to best reflect changes by ablation in a low number of features.

In a preferred embodiment according to claim 12 the assignment of channels to groups is determined by the control system and/or input by a user. This allows flexible configuring the groups to match the position of the catheter respectively the electrodes.

Claim 1 relates to the ultimate decision-making based on the feature space. The feature space may be used to arrive a prognosis about the success of an ablation therapy and/or to derive a proposal for a next step of the ablation therapy. The control system may for example use an expert model fed with experience of physicians to aid the physician in deriving a therapy.

A very preferred embodiment of claim 13 relates to using a trained AI-model fed with the feature space. Generally, getting good results from an AI in the medical field, in particular in the field of atrial fibrillation, is difficult as many parameters relating to for example the complexity of the atrial fibrillation and generally describing the patient are not available in a structured data format or not available at all. Further, even if 3D-mapping-systems are able to measure a lot of data, this data is very specific to the anatomy of each heart and can therefore hardly be used for an AI or at least poses a very complex problem for the AI. It is a challenge to design an AI with a well-defined problem which can be solved by a state of the art AI. The question whether an AI is able to provide good output depends on the definition of the input data, the overall complexity of the problem, the definition of the AI-model and the chosen output data. Here, it is proposed to use a specific set of input data, the feature space, which is optimized to provide a homogenous overview of changes during ablation therapy without including too much data. The use of the feature space as input for an AI-model therefore significantly reduces the complexity of the problem and therefore improves the output of the AI-model. The proposed prognosis about the success of the ablation therapy, the proposal of a next step or the prognosis of a change of the feature space as output of the AI-model are directly connected to the feature space and therefore pose well-defined problems to solve by AI.

Another teaching according to claim 14, which is of equal importance, relates to a control system for analysing an intracardiac electrogram, in particular configured to perform the proposed method, wherein the intracardiac electrogram has been recorded via a catheter inserted into a human body and has a time length, wherein the catheter comprises multiple electrodes placed at different anatomical positions, wherein multiple channels of the intracardiac electrogram have been recorded by the electrodes, wherein the control system groups the channels into at least a first group of channels comprising at least one channel assigned to a first anatomical region and a second group of channels comprising at least one channel assigned to a second anatomical region, wherein the control system derives derived features comprising a first derived feature relating to the first anatomical region and a different second derived feature relating to the first anatomical region from the first group of channels and derives the first derived feature and the second derived feature relating to a second anatomical region from the second group of channels, wherein data points of the derived features are derived in intervals over the time length, the first and second features thereby each having a time dimension and a value dimension with a value range, wherein the control system combines the derived features into a feature space comprising at least an anatomical dimension and a feature dimension and a time dimension and an intensity dimension, wherein the anatomical dimension comprises the anatomical regions, wherein the feature dimension comprises the derived features, wherein the time dimension is the time dimension of the first and the second derived features, wherein the intensity dimension comprises an intensity range and wherein the control system transforms the value ranges of the derived features onto the intensity range.

All explanations given with regard to the proposed method are fully applicable.

In an embodiment according to claim 15 the control system may comprise an interface to a measurement system and/or to the catheter and may be configured to be used during ablation therapy.

In the following, embodiments of the invention are explained with respect to the drawing. The drawing shows in

Fig. 1,    a human heart, two catheters and a control system during an exemplary surgical procedure,

Fig. 2,    a display with the feature space, a surface ECG and an intracardiac electrogram in that order from top to bottom,

Fig. 3,    deriving derived features in time intervals and

Fig. 4,      the displaying of the feature space in more detail and a further embodiment.

The proposed method concerns the treatment and analysis of atrial fibrillation. Of particular interest is the treatment of atrial fibrillation (AF) by ablation therapy, in particular by stepwise ablation therapy. The stepwise ablation therapy comprises a number of ablation targets and an ablation procedure for each target. An exemplary stepwise ablation might comprise ablating the pulmonary veins and then other targets such as focal points identified through a 3D mapping system.

During the stepwise ablation, the physician must decide whether to continue with the steps of the ablation therapy or stop. A usual stopping point is the reversion to a sinus rhythm of the patient. However, stepwise ablation often needs to be performed more than once for a patient to reach a lasting therapeutic effect, namely no recurrence of AF.

The proposed method and control system 1 allow a physician to gain a better understanding of what happens inside a patient's heart, electrically.

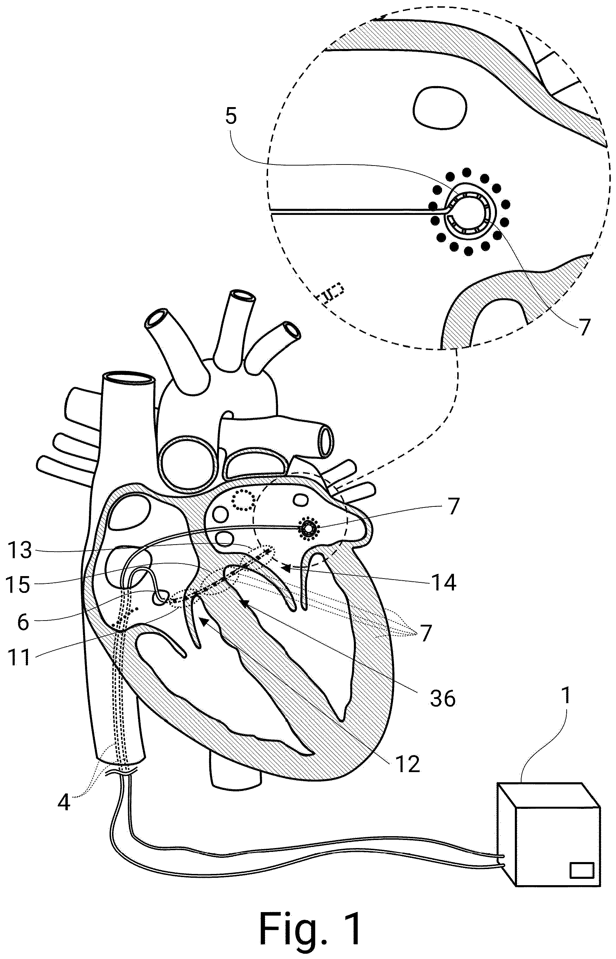

Proposed is a method for analysing an intracardiac electrogram 2 via a control system 1. The control system 1 may be a local unit with a processor, possibly a display 3 and the like. It may be a general purpose PC or a dedicated system. Fig. 1 shows schematically a heart and two diagnostic catheters 4 entered into the heart from the femoral vein. The circular catheter 5 is placed in a pulmonary vein, the decapolar catheter 6 is placed in a vein at the back of the heart, preferably the coronary sinus. The control system 1 is shown as a box connected to the catheters 4 but may just as well be a system connected to a measurement system and only indirectly receiving the intracardiac electrogram 2. Preferably, the control system 1 is used during surgery. An ablation catheter 4 may also be present but is not shown.

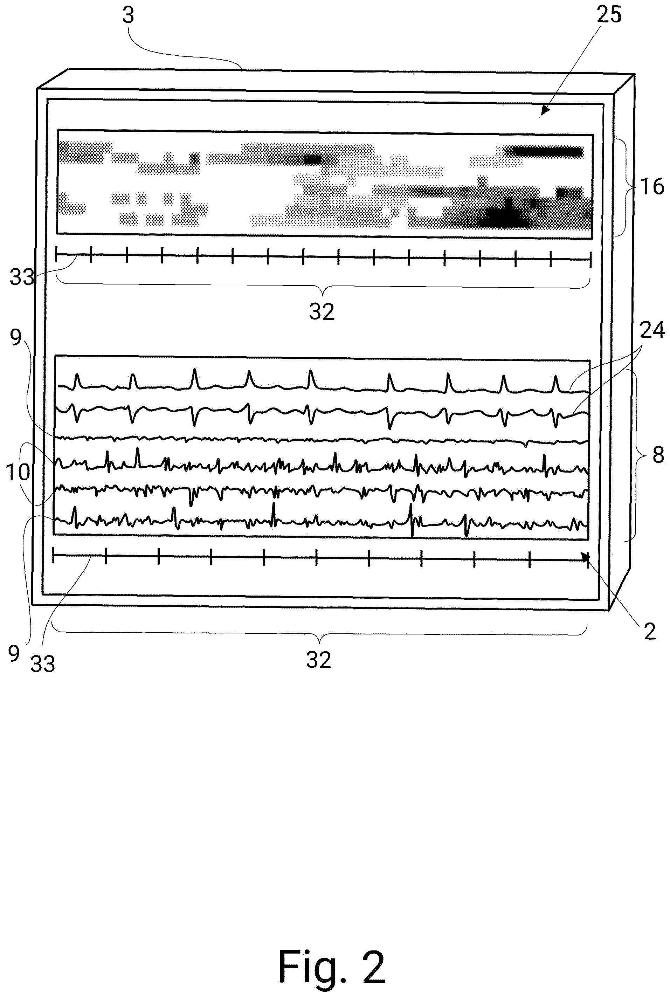

The intracardiac electrogram 2 has been recorded, in particular during ablation therapy, via a catheter 4 inserted into a human body and has a time length. The electrogram may be updated in real time during surgery. The time length of the intracardiac electrogram 2 may be for example 10 minutes. New data may be appended at the end or the length may be constant and new data may overwrite old data. For the purpose of the present method, the overall length of the intracardiac electrogram 2 is not relevant. It only needs to have a certain minimum length for the calculations to be possible. Fig. 2 shows in the bottom view a section of an intracardiac electrogram 2.

The catheter 4 comprises multiple electrodes 7 placed at different anatomical positions. The anatomical positions do not have to be defined or even known to the control system 1. It will be explained how, optionally, some knowledge about the anatomical positions can be advantageous and inferred from the type of ablation procedure, for example. Fig. 1 shows how the ten electrodes 7 of the decapolar catheter 6 may extend from the right atrium (left side of Fig. 1) to the left atrium (right side of Fig. 1).

Multiple channels 8 of the intracardiac electrogram 2 have been recorded by the electrodes 7. Fig. 2 shows four channels 8 of the intracardiac electrogram 2 (bottom four rows). The top channel of the four channels and the bottom channel of the four channels are unipolar channels 9, the two in the middle are bipolar channels 10. The unipolar channels 9 and bipolar channels 10 may be measured by the same catheter 4. By not displaying all channels 8 of the intracardiac electrogram 2 a more compact overview can be achieved. The example of Fig. 2 therefore further emphasizes that the proposed method aims at giving the physician an imprecise but more global overview of the ablation therapy. The reasoning behind this broader overview is that an ablation therapy and its effects cannot be captured in precise absolute measurement values due to the inherent intra-and interperson variability.

The control system 1 groups the channels 8 into at least a first group 11 of channels 8 comprising at least one channel 8 assigned to a first anatomical region 12 and a second group 13 of channels 8 comprising at least one channel 8 assigned to a second anatomical region 14. Fig. 1 shows a first group 11 of channels 8, a second group 13 of channels 8 and a yet to be described third group 15 of channels 8 on the decapolar catheter 6. The first group 11 of channels 8 comprises three electrodes 7 assigned to the right atrium, the second group 13 of channels 8 comprises three electrodes 7 assigned to the left atrium and the third group 15 of channels 8 comprises four electrodes 7 not clearly assigned to either atrium and therefore assigned to a transitional anatomical region. Depending on whether unipolar channels 9 and/or bipolar channels 10 are used, the number of channels 8 assigned to a respective group in this case can vary.

An anatomical region may be any location in the heart. Preferably, the anatomical regions that the channels 8 of the first group 11 and/or second group 13 is or are assigned to is an anatomical structure. Preferably, the channels 8 of the first group 11 and the channels 8 of the second group 13 are assigned to different anatomical structures. An anatomical structure is an electrically and functionally delimited region. For present purposes the heart comprises four anatomical structures, namely the atria and the ventricles. There may be other anatomical structures like a pulmonary vein associated to the heart and the heart does comprise anatomical structures which are not part of the atria and ventricles and not in focus of the present method but generally comprised by the term. In the example of Fig. 1, the first group 11 and the second group 13 are assigned to different anatomical structures and the third group 15 is not assigned to an anatomical structure.

The control system 1 does not need to know the assignment of the groups. The physician may know how to use and interpret an output of the control system 1 for a certain procedure.

In one embodiment, the anatomical positions are known to the control system 1, in particular from a 3D mapping system. The control system 1 may be part of a 3D mapping system or may be a 3D mapping system. In particular if the control system 1 has some knowledge about the anatomical positions, the assignment of channels 8 to groups may be automatically done based on a known position of one or more electrodes 7. Additionally or alternatively, a user may be involved in assigning the channels 8 to the groups. In particular, the control system may visualize a possibly assignment, for example by highlighting positions of electrodes 7 belonging to channels 8 such that the user can easier choose or corroborate the assignment.

Here and preferably, at least one channel 8 of one group is assigned to the left atrium and at least one channel 8 of another group is assigned to the right atrium. Additionally or alternatively, at least one channel 8 of at least one group is assigned to a group, by the control system 1 and/or by the user, based on its anatomical position known to the control system 1.

The control system 1 derives derived features 16 comprising a first derived feature 17 relating to the first anatomical region 12 and a different second derived feature 18 relating to the first anatomical region 12 from the first group 11 of channels 8 and derives the first derived feature 17 and the second derived feature 18 relating to a second anatomical region 14 from the second group 13 of channels 8.

Derived features 16 are features derived from one or more channels 8 by processing the information of the channels 8. Derived features 16 can be time-aggregated features like a heart rate, a heart rate variability, different intervals 19, in particular a beat-to-beat time, and the like. The first derived feature 17 and the second derived feature 18 are derived each for the first group 11 and for the second group 13 from one or more, preferably all, channels 8 of the respective group. Here, that means that there are at least four derived features 16, namely the first derived feature 17 and the second derived feature 18 are derived at least twice, once for each group. The derived features 16 are here and preferably derived independently for each group.

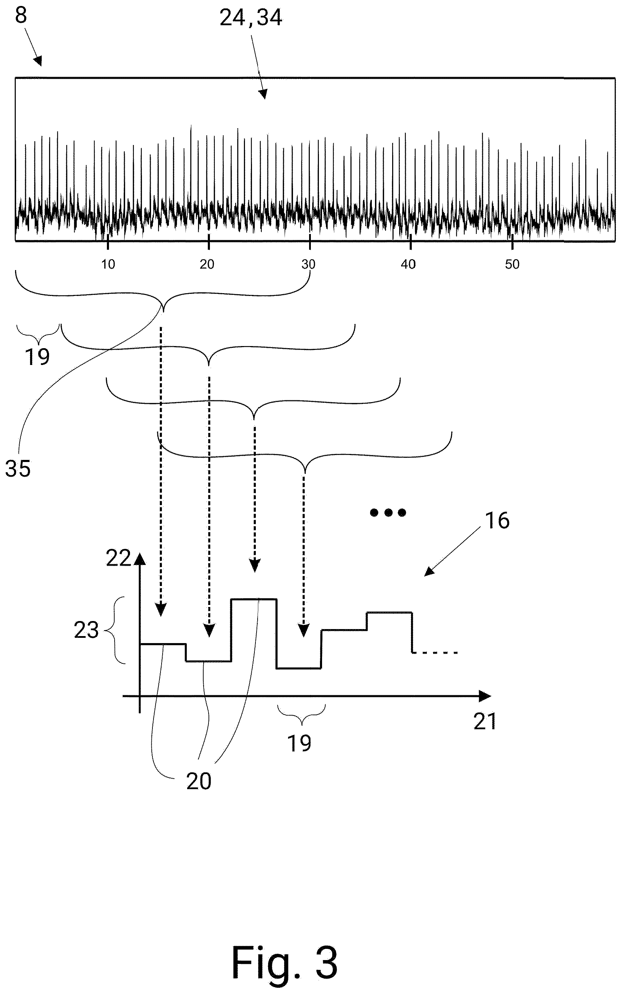

The control system 1 derives data points 20 of the derived features 16 in intervals 19 over the time length, the first and second features thereby each having a time dimension 21 and a value dimension 22 with a value range 23. Fig. 3 shows 60 seconds of an exemplary channel 8, in this case of a surface ECG 24, that are processed into a derived feature (bottom of Fig. 3). The interval 19 in Fig. 3 is five seconds, therefore each five seconds a data point 20 of the derived feature is generated from the last 30 seconds of data of the channel 8. Here and preferably the intervals 19 are greater than a sampling time such that the derived features 16 are time-aggregated. The intervals 19 define the update-cycle of the derived features 16.

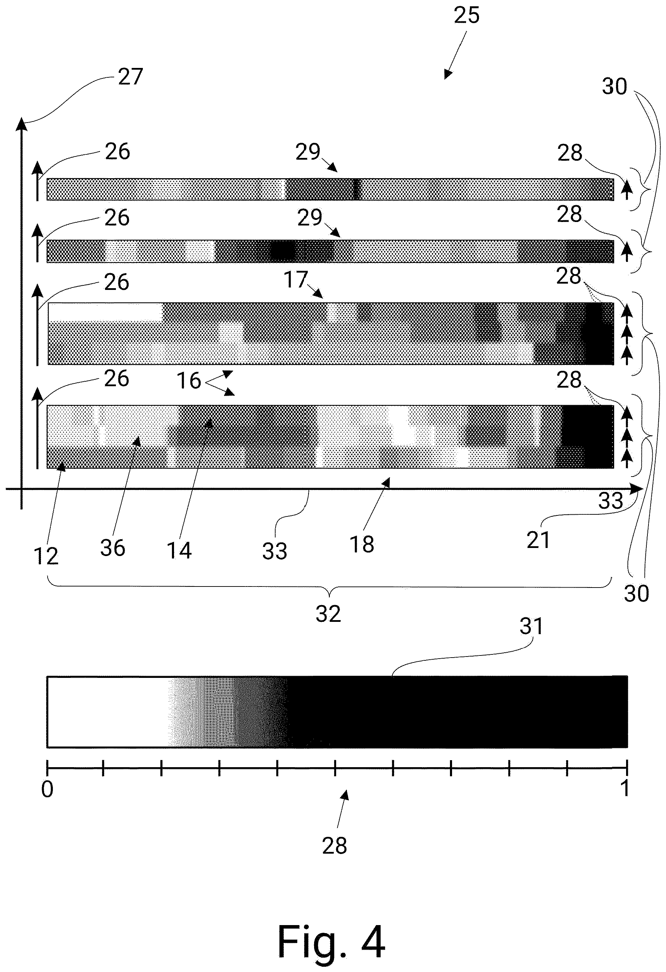

The control system 1 combines the derived features 16 into a feature space 25 comprising at least an anatomical dimension 26 and a feature dimension 27 and a time dimension 21 and an intensity dimension 28. Fig. 4 shows a visualization of the feature space 25. The intensity dimension 28 is displayed by colors of a heatmap shown in a grey scale here. Fig. 2 shows a different version of the feature space 25 displayed on a display 3 of the control system 1. The visualization on the display 3 in Fig. 2 may as well be the version of Fig. 4 in another embodiment. Fig. 4 shows two derived features 16 for three groups of the decapolar catheter 6 and two surface features 29, which are also derived features 16 but from a surface ECG 24, that will be explained later.

Focusing on the lower six rows of the feature space 25 in Fig. 4, it can be seen that they are visually grouped into two groups. Each of those visual groups 30 represents one feature. Each of the visual groups 30 contains three rows, one for every group of channels 8. For example, the lowest three rows of the feature space 25 of Fig. 4 are a row for the right atrium (first group 11), a row for the transitional group (third group 15) and a row for the left atrium (second group 13). It can therefore easily be compared how the feature behaves for the different anatomical regions. It is further easy to see how the anatomical regions behave over multiple derived features 16 by comparing, for example, the first rows of both visual groups 30. Of course, further visual groups 30 may be present for other derived features 16.

Turning to the definition of the feature space 25, the anatomical dimension 26 comprises the anatomical regions, the feature dimension 27 comprises the derived features 16, the time dimension 21 is the time dimension 21 of the first and the second derived features 16 and the intensity dimension 28 comprises an intensity range 31.

The anatomical dimension 26 is a discontinuous dimension which may consist of only the first anatomical region 12 and the second anatomical region 14. The feature dimension 27 is also a discontinuous dimension comprising a number of at least two features.

The time dimension 21 comprises the time dimensions 21 of all features, which are preferably the same or substantially overlap. Here and preferably, the time dimensions 21 of the features overlap for at least 50%, more preferably at least 90%. For easy calculation it is preferred that all data points 20 of all features are equally spaced and located at the same points in time. Nevertheless, the data points 20 of different features could also have a certain offset or the like. Fig. 3 shows how a time dimension 21 of one feature may be defined. In Figs. 2 and 4 the features all have the same time dimension 21 with the same time length and over the same absolute time range, for example the last 15 minutes.

The derived features 16 may have and have preferably very different value ranges 23 of intensity values, in particular even with different units. Proposed is now that the control system 1 transforms the value ranges 23 of the derived features 16 onto the intensity range 31.

The resulting intensity range 31 of the feature space 25 may for example consist of the range from 0 to 1. The intensity range 31 may be displayed by a color range from blue to red, for example with blue being 0 and red 1 or vice versa.

The proposed deriving of features for different anatomical regions and transforming them into a single feature space 25 is presently focused on two main applications that can be subsumed under "better decision-making". The decisions may either be made by a human, in particular a physician, and/or by an automated system, in particular an AI system.

AI systems are strongly dependent on their input data. Trying to make an AI learn on its own, through training, that input data may be grouped into anatomical regions and that the absolute values are not well usable for deriving a therapy prognosis is a big challenge compared with providing an AI system with a feature space 25 that fits the application already by its formatting and by choosing relevant data and deliberately excluding irrelevant data.

A human operator may be able to find logical groupings quicker and use their experience. However, a human operator does not have signal processing capabilities to derive features from ECGs. Therefore, the selection of a fitting abstraction of data enables a human operator to understand more about the internals of the patient and derive conclusions.

The present invention focuses on providing an improved level of overview about the relative changes during ablation therapy and the electrical and physiological reactions by the heart of the patient and deliberately forgoes accuracy, in particular regarding absolute values. This results in a better understandability of the digital representation of biological processes, be it by a machine or by a human.

The actual values of the derived features 16 after transformation may not always use the full intensity range 31. However, the intensity range 31 is chosen such that the general comparability between the intensity of different features is given. Therefore, throughout a procedure, the parts of the intensity range 31 used by the transformed derived features 16 will normally significantly overlap. As Fig. 2 shows in the left part of the feature space 25, at the beginning of the procedure, exemplarily, all features may be less intense than at the end, thereby showing that the procedure had a significant effect somewhere in the middle and somewhere in the last third of the displayed time length 32.

It is important to note that the transformation functions for the different features will naturally differ. In particular if the units of the derived features 16 are different, the transformations functions will significantly differ. If the units are equal, in particular if the features are unitless, prior to the transformation their value ranges 23 will also be different, preferably not overlapping.

It may be the case that the intracardiac electrogram 2 comprises only channels 8 recorded by a single catheter 4, however, as shown, multiple catheters 4 may be used. In that case, preferably, a channel 8 is defined by one or two electrodes 7 of a single catheter 4. It may be the case that at least two of the groups include only channels 8 of a single catheter 4. It may also be the case, that at least two of the groups include channels 8 from different catheters 4, whereby in particular each of the at least two groups includes only channels 8 of a single catheter 4.

The assignment of channels 8 to groups may be changed over time, in particular automatically by the control system 1. In particular, the assignment of channels 8 to groups may be changed after a repositioning of the catheter 4. Preferably, a resulting map of the heart has a varying number of channels for at least some anatomical regions. In the case of a map of the heart, the anatomical region may be the smallest unit displayed in the anatomical dimension 26. For example, each anatomical region may have a single color at a certain point in time.

The assignment of one or more channels 8 to the groups may be predefined or derived from the control system 1. In particular, it may be assigned based on the order of electrodes 7 or channels 8 on the catheter 4. In particular, the first group 11 may comprise the most proximal channel 8 of the intracardiac electrogram 2 and/or the second group 13 may comprise the most distal channel 8 of the intracardiac electrogram 2. The assignment may be chosen by the control system 1 based on knowledge about the procedure. The knowledge may be derived from measurements or input by a user, for example by choosing a procedure on an interface.

According to one embodiment it is proposed, that the control system 1 comprises a display 3 and that the control system 1 displays on the display 3 the at least, preferably exactly, four dimensions of the feature space 25. Figs. 2 and 4 show how this can be realized. Fig. 4 shows a time dimension 21 from left to right, a feature dimension 27 from bottom to top and inside the feature dimension 27 the anatomical dimension 26 by visual grouping of features such that different features are visually delimited. The arrows on the right in Fig. 4 are a representation of the color values of the intensity dimension 28 of each row.

If the control system 1 has knowledge about the anatomical positions, it may be the case that the anatomical dimension 26 is displayed as a representation of the heart, in particular a 3D representation of the heart. One or more of the other dimensions may then be displayed related to the anatomical position.

As has been mentioned, one interesting embodiment of the proposed solution is the time aggregation of the derived features 16 to allow a broader overview of changes over time. It is preferred, that the intervals 19 are at least 0.5 seconds long, preferably at least 1 second long, more preferably at least two seconds long and that the control system 1 displays at least 5 minutes, preferably at least 8 minutes, more preferably at least 12 minutes, of the time dimension 21. The displayed part of the time dimension 21 is displayed at the same time, in particular without scrolling. Further data may be available with scrolling, for example up to 30 minutes or up to 45 minutes. Here and preferably, the sampling frequency of the intracardiac electrogram 2 is such that the intervals 19 are much longer than the time between sampling points and therefore the aggregation of data over time is substantial. The sampling frequency may be in the range of kHz. It is preferred that the control system 1 displays on the display 3 the at least, preferably exactly, four dimensions of the feature space 25 at the same time, in particular without scrolling.

Another preferred way that the control system 1 may display on the display 3 the four dimensions of the feature space 25 is that the control system 1 displays the anatomical dimension 26 and the feature dimension 27 and the intensity dimension 28 at the same time without scrolling and the time dimension 21 with slicing. For example, one or more 3D maps of the heart may show the anatomical dimension 26 by placing the intensity values at anatomical positions on the map. One or more features may be displayed at the same position or next to each other for each anatomical region. The time dimension 21 may be displayed by slicing through time such that intensity values of different points in time are not displayed at the same time. Different than other mapping systems however, for the different anatomical regions, a time dimension actually exists. Other mapping systems assume that there are no changes over time.

The intervals 19 are preferably smaller than or equal to 10 seconds. The displayed time dimension 21 is preferably smaller than or equal to 1.5 hours, preferably 45 minutes, more preferably 30 minutes.

It is further preferred that the control system 1 displays on the display 3 several rows of data along a common time axis 33. Rows may be horizontally arranged or vertically arranged. The rows of data comprise a row for each of the derived features 16. The rows comprise the data points 20 of the respective feature along the time axis 33 and mapped intensity values of the intensity range 31 for the data points 20. Preferably, the intensity values are color coded, in particular as a heatmap.

It is further possible to display 3 the moments in time at which an ablation was performed. That makes it even easier to spot a reaction to a certain ablation event. It may be possible for a user to select which features of the feature dimension 27 should be displayed.

The rows of data may comprise a row for the first derived feature 17 of the first group 11, a row for the second derived feature 18 of the first group 11, a row for the first derived feature 17 of the second group 13 and a row for the second derived feature 18 of the second group 13.

According to one embodiment it is proposed, that the control system 1 low pass filters the intensity values of the derived features 16 along the time dimension 21 and displays the low passed derived features 16, and/or, that the control system 1 visually groups the derived features 16 along the feature dimension 27 and displays the anatomical dimension 26 of each feature as a visual group 30 along the anatomical dimension 26. Alternatively, the control system 1 may visually group the features along the anatomical dimension 26 thereby displaying the features of the first group 11 in a visual group 30 and the features of the second group 13 in a visual group 30. This last embodiment is not shown in the figures.

A possibility is that the control system 1 further displays on the display 3 at least one channel 8 of a surface ECG 24 (Fig. 2). Preferably, the control system 1 displays on the display 3 a section of the at least one channel 8 of the surface ECG 24 and/or at least one channel 8 of the intracardiac electrogram 2 with a displayed time length 32 shorter than the displayed time length 32 of the time dimension 21 of the feature space 25 and/or with data points 20, in particular ECG data points 34, spaced apart less than the length of the intervals 19.

Preferably, the displayed time length 32 of the channel 8 is less than 1 minute, preferably less than 30 seconds, and/or, the data points 20 are spaced apart less than 100 ms, preferably less than 10 ms, more preferably at most 1 ms. In Fig. 2, it is 10 seconds. As has been mentioned, the ECG sampling frequency may be in the kHz range, resulting in data points 20 spaced apart less than 1 ms of a second.

The control system 1 may be connected to electrodes of the surface ECG 24 or to a system measuring the surface ECG 24.

According to one embodiment it is proposed, that the data points 20 of the derived features 16 are calculated over time spans 35 greater than the intervals 19, preferably, that the time spans 35 are at least ten seconds, preferably at least 20 seconds, and/or less than one minute, preferably less than 45 seconds. As Fig. 3 shows, a time span 35 may overlap multiple intervals 19.

To enhance the overview of the state of the electrical heart, it may be the case that the control system 1 further derives at least one, preferably at least two, surface features 29 from the surface ECG 24, that the surface features 29 have a time dimension 21 and a value dimension 22, that the control system 1 inserts the surface features 29 into the feature space 25 such that the feature dimension 27 comprises the surface features 29, that the anatomical dimension 26 comprises a global anatomical region, that the time dimension 21 of the surface features 29 is mapped onto the time dimension 21 of the feature space 25 and that the control system 1 transforms the value ranges 23 of the value dimension 22 of the surface features 29 onto the intensity range 31.

It is interesting to note that the surface ECG 24 features may be derived with different intervals 19 and/or over different time spans 35 than the derived features 16 of the intracardiac electrogram 2 and naturally have different value ranges 23. Still, they can be included in the same feature space 25 because the focus of the proposed method is on a global overview as explained. Surface features 29 mostly relate to the heart as one global anatomical region even though for example by a P-wave-analysis, the atria can be focused.

If the plural is used behind the explanation of one or more aspects, it may also stand for the singular and is used for better readability.

Here and preferably, the control system 1 constantly receives new data from a measurement system comprising at least the catheter 4 and updates the feature space 25. Preferably, the control system 1 updates the display 3 periodically, in particular in real time.

The measurement system may comprise hardware for measuring, analog or digital filters, etc. and may comprise or be connected to a further control system 1, for example a 3D mapping system. Alternatively, the control system 1 may be connected directly to the catheters 4.

It is proposed, that the control system 1 transforms the value ranges 23 of the derived features 16 onto the intensity range 31 via a different transformation function per feature, preferably, that the transformation function is equal for all anatomical regions per feature. Providing substantially the same transformation function for a feature at different anatomical regions enables an intra-feature comparison. It becomes possible to see how a feature changes in particular differently between the left and right atria.

Turning now to the details of the transformation function, it may be the case that at least one, preferably all, transformation functions are non-linear. A linear normalization may typically define a min and max value such that

$$f(x) = (x - mean(x))/(max(x)-min(x))$$

I.e., f(x) will now have values between 0 and 1 and has a linear relation to the values of x. x would be the value dimension 22 of the derived features 16 and f(x) the intensity dimension 28.

Here, it may be the case that a non-linear expression is used as "f(x)". This allows for compression or decompression of the scale that may increase sensitivity of the normalized value to a specific range of input-values. Examples of non-linear transforms are exponentials, logarithms, sigmoid and piece-wise linear functions.

Additionally or alternatively, the transformation functions are updated based on the value ranges 23 of the respective feature or of all anatomical regions per feature over the time dimension 21 or part of the time dimension 21, in particular the displayed time dimension 21. Preferably, the part of the time dimension 21 changes with new intervals 19, in particular with every new interval 19.

The proposed transformation varies over time as the "significant change" depends on the data. An adaptive transformation where the transformation coefficients are derived from the data continuously rather than being fixed may be used. This means that in a particularly preferred embodiment, the values already displayed on the display 3 may be updated and therefore changed based on a new transformation. It may be counter-intuitive to change values after displaying them but helps the proposed target of showing significant changes. Possibly, a change of already displayed values may be performed only if certain threshold is met.

One transformation algorithm may use the mean and standard variation to make a statistical normalization, for example with or based on f(x) = (x-mean(x))/std(x).

In one specific solution, the most recent 15 minutes of data may be used to define the transformation.

Further it may be the case that the control system 1 adapts at least one of the transformation functions, preferably all the transformation functions, based on the value range 23 of the respective feature or feature group, in particular over the whole time dimension 21 or part of the time dimension 21, in particular the displayed part of the time dimension 21, and based on predefined boundaries, in particular boundaries for the center of the transformation function.

If a physiological beat-to-beat time is known to be around 1 second, the midpoint of a transformation function, linear or not, may be forced to be between 0.9 seconds and 1.1 seconds. A minimum beat-to-beat time of 0.8 seconds with the maximum below 0.9 seconds would then define, in a linear case, the transformation of 0.8 to 1 seconds onto the intensity range 31 of 0 to 1 with 0.8 seconds transformed to 0 and 1 second transformed to 1. The midpoint forced to 0.9 seconds would be transformed to 0.5. Similar examples can be found for other features. This example would lead to the heatmap of the beat-to-beat time to show mainly blue values. The boundaries may be predefined based on physiological knowledge.

It may also be the case that the control system 1 adapts at least one of the transformation functions, preferably all the transformation functions, such the transformation of the values of some data points 20 of the respective feature depends on the values of data points 20 located later in time. Often, real-time calculations do not change the past based on new updates. It is proposed to do exactly that to better visualize significant changes. Instead of predicting a good transformation function and possibly not catching the significant change, it is accepted that previous values may change to better capture significant changes based on actual changes. It may there be the case that a transformation function is applied to historic data points 20 which is derived from more recent data points 20. The transformation function may be derived from the more recent data points 20 and all data points 20 it is applied to or there may be data points 20 that the transformation function is applied to which are not used to derive the transformation function.

Here and preferably, the transformation of some data points 20, in particular all data points 20 minus one per feature, depends on data points 20 later in time.

According to one embodiment it is proposed, that the first group 11 and the second group 13 each comprise at least two channels 8, and/or, that the control system 1 further groups the channels 8 into at least a third group 15 of channels 8 comprising at least one, preferably at least two, channels 8 assigned to a third anatomical region 36.

As Fig. 1 shows, several electrodes 7 may be part of one group. While it is known to average multiple channels 8 to reduce noise, here, it is proposed to base that averaging on the anatomical location of the electrodes 7 and therefore channels 8. Averaging a channel 8 of the left atrium with a channel 8 of the right atrium may actually remove a lot of relevant information. Averaging or otherwise combining the channels 8 of the left atrium only however can increase the precision, decrease noise and decrease the overall information in the feature space 25. The third group 15 may be a transitional group between the left and right atria.

Preferably, the control system 1 combines at least two, preferably all, channels 8 of a, preferably every, group, in particular by calculating an average, into a single data point 20 per feature per time interval 19.

It may be the case that two groups of the three groups comprise exactly three channels 8 and/or that one group of the three groups comprises exactly four channels 8.

Here and preferably, a single derived feature per feature is derived per group, meaning that for example the first derived feature 17 is calculated once for the first group 11 by combining for example three channels 8 of the first group 11 into the one derived feature instead of calculating the derived feature for each channel 8.

The term "average" comprises the mean, the median, etc.

Turning to the features of the feature dimension 27, it may be the case that at least one of the features, preferably at least two, more preferably at least three, of the features of the feature dimension 27, are chosen from the group consisting of dominant frequency, autocorrelation of the surface ECG 24, a cross correlation of the intracardiac electrogram 2, cycle length, local activation time dispersion, fractionation index, cycle length variation, dominant frequency gradient. Ways of calculating the named features are known in the art. Which exact way is used is open to different embodiments.

According to one embodiment it is proposed, that the control system 1 determines the assignment of channels 8 to groups based on measured data of the intracardiac electrogram 2, in particular once per procedure, and/or, that a user may assign a subset of the channels 8 to a group. The control system 1 may "guess" the performed ablation therapy, in particular the location of the catheter 4 from knowledge about usual proceedings and may display 3 this "guess" to the user. The catheter 4 itself may be placed by the physician based on ultrasound images for example without the control system 1 knowing about the details. The control system 1 may automatically detect similarities and differences between channels 8 and determine the assignment to groups based on similar signals in channels 8 and in particular additionally based on the order of electrodes 7 on the catheter 4.

Turning now towards the automated decision-making, it may be the case that the control system 1 uses the feature space 25 to derive a prognosis about the success of the ablation therapy. The control system 1 may use a trained AI model to derive the prognosis. The trained AI model may receive the feature space 25 as input data and be trained to detect a significant change that is related to a success in the ablation therapy. Success may mean that AF does recur. By feeding the AI with real time updated of the feature space 25, the AI receives varying data adapted to best represent significant changes. The training of the AI model may therefore have been focused on detecting which changes are significant and related to therapy success. This problem is much more well-defined than for example inputting the intracardiac electrogram 2 into an AI.

Additionally or alternatively, the control system 1 uses the feature space 25 to derive a proposal for a next step in the ablation therapy. The next step may be one of a few possible ablation steps, in particular including an ablation location. The AI model may then be trained to recognize electrical situations that may potentially change significantly by an ablation therapy. In particular, the AI model may be trained to predict a change in the feature space 25 by a given ablation step. It may therefore be the case that the control system 1 uses the feature space 25 to derive a prognosis of the change of the feature space 25 by a possible ablation step,

Training data of the AI may have included feature spaces 25 with significant changes related to therapy success. As the feature spaces 25 are less related to the specific anatomy and pathology of a patient, more general training data can be made available by using the proposed feature space 25. The number of different possible ablation steps may be low, for example less than five. The AI may then be used by the physician to decide which of the low number of possible ablation steps to try next. Previously, the order of steps was either predefined or at the discretion of the physician. A possible step may also be not performing another step.

The AI models are preferably used by the control system 1 by inputting the feature space 25 into a trained AI model.

The AI may have been trained by supervised learning, unsupervised learning, deep learning or reinforcement learning. Supervised learning is a type of AI that uses labeled data to train a model to predict outcomes or classify data points 20. It is used to analyse a feature space 25 and derive a prognosis by using labeled data points 20 to learn a model that can be used to make predictions. The labels may include the treatment success after for example a year.

Unsupervised learning is a type of AI that uses unlabeled data to group similar data points 20 together. It can be used to analyse a feature space 25 and derive a prognosis by clustering data points 20 into separate groups and analysing the characteristics of each group. Particularly, unsupervised learning may be used to derive the prognosis about the change in the feature space 25.

Deep learning is a type of AI that uses large neural networks to process data and make predictions. It can be used to analyse a feature space 25 and derive a prognosis by learning complex relationships between different features. Here, deep learning may be helpful to sufficiently use the proposed anatomical grouping.

Reinforcement learning is a type of AI that uses rewards and punishments to teach a model how to behave. It can be used to analyse a feature space 25 and derive a prognosis by teaching a model how to make decisions in order to achieve the best outcome. The model may be rewarded if a simulated patient does not recur and punished, if another treatment was necessary.

Generally, databases with ablation proceedings may be used to train the AI model. The advantage of providing an AI with a well-defined feature space 25 of derived features 16 instead of raw data is that it can more easily identify patterns, reduce noise, and identify meaningful correlations. By providing the AI with a well-defined feature space 25, the AI can focus on the most meaningful features and learn from them more effectively. This helps reduce the amount of data the AI needs to process and makes the learning process more efficient. Additionally, it can help provide more accurate prognoses since the AI can focus on the most relevant features.

Another teaching which is of equal importance relates to a control system 1 for analysing an intracardiac electrogram 2, in particular configured to perform the proposed method, wherein the intracardiac electrogram 2 has been recorded via a catheter 4 inserted into a human body and has a time length, wherein the catheter 4 comprises multiple electrodes 7 placed at different anatomical positions, wherein multiple channels 8 of the intracardiac electrogram 2 have been recorded by the electrodes 7, wherein the control system 1 groups the channels 8 into at least a first group 11 of channels 8 comprising at least one channel 8 assigned to a first anatomical region 12 and a second group 13 of channels 8 comprising at least one channel 8 assigned to a second anatomical region 14, wherein the control system 1 derives derived features 16 comprising a first derived feature 17 relating to the first anatomical region 12 and a different second derived feature 18 relating to the first anatomical region 12 from the first group 11 of channels 8 and derives the first derived feature 17 and the second derived feature 18 relating to a second anatomical region 14 from the second group 13 of channels 8, wherein data points 20 of the derived features 16 are derived in intervals 19 over the time length, the first and second features thereby each having a time dimension 21 and a value dimension 22 with a value range 23, wherein the control system 1 combines the derived features 16 into a feature space 25 comprising at least an anatomical dimension 26 and a feature dimension 27 and a time dimension 21 and an intensity dimension 28, wherein the anatomical dimension 26 comprises the anatomical regions, wherein the feature dimension 27 comprises the derived features 16, wherein the time dimension 21 is the time dimension 21 of the first and the second derived features 16, wherein the intensity dimension 28 comprises an intensity range 31 and wherein the control system 1 transforms the value ranges 23 of the derived features 16 onto the intensity range 31.

The intracardiac electrogram 2 was provided to the control system 1 and is preferably regularly updated. The control system 1 preferably comprises the display 3.

According to one embodiment it is proposed, that the control system 1 comprises an interface to a measurement system and/or to the catheter 4 and is configured to be used during ablation therapy, in particular stepwise ablation therapy, to aid in determining a prognosis about the success of the ablation therapy.

**Reference numbers**

| | |
|---|---|
| 1 | control system |
| 2 | intracardiac electrogram |
| 3 | display |
| 4 | catheter |
| 5 | circular catheter |
| 6 | decapolar catheter |
| 7 | electrode |
| 8 | channel |
| 9 | unipolar channel |
| 10 | bipolar channel |
| 11 | first group |
| 12 | first anatomical region |
| 13 | second group |
| 14 | second anatomical region |
| 15 | third group |
| 16 | derived features |
| 17 | first derived feature |
| 18 | second derived feature |
| 19 | interval |
| 20 | data point |
| 21 | time dimension |
| 22 | value dimension |
| 23 | value range |
| 24 | surface ECG |
| 25 | feature space |
| 26 | anatomical dimension |
| 27 | feature dimension |
| 28 | intensity dimension |

29    surface feature
30    visual group
31    intensity range
32    displayed time length
33    time axis
34    ECG data points
35    time span
36    third anatomical region

**Claims**

1. Method for analysing an intracardiac electrogram (2) via a control system (1), wherein the intracardiac electrogram (2) has been recorded, in particular during ablation therapy, via a catheter (4) inserted into a human body and has a time length, wherein the catheter (4) comprises multiple electrodes (7) placed at different anatomical positions, wherein multiple channels (8) of the intracardiac electrogram (2) have been recorded by the electrodes (7), wherein the control system (1) groups the channels (8) into at least a first group (11) of channels (8) comprising at least one channel (8) assigned to a first anatomical region (12) and a second group (13) of channels (8) comprising at least one channel (8) assigned to a second anatomical region (14),

wherein the control system (1) derives derived features (16) comprising a first derived feature (17) relating to the first anatomical region (12) and a different second derived feature (18) relating to the first anatomical region (12) from the first group (11) of channels (8) and derives the first derived feature (17) and the second derived feature (18) relating to a second anatomical region (14) from the second group (13) of channels (8),
wherein the control system (1) derives data points (20) of the derived features (16) in intervals (19) over the time length, the first and second features thereby each having a time dimension (21) and a value dimension (22) with a value range (23),
**characterized in that** the control system (1) combines the derived features (16) into a feature space (25) comprising at least an anatomical dimension (26) and a feature dimension (27) and a time dimension (21) and an intensity dimension (28), that the anatomical dimension (26) comprises the anatomical regions, that the feature dimension (27) comprises the derived features (16), that the time dimension (21) is the time dimension (21) of the first and the second derived features (16), that the intensity dimension (28) comprises an intensity range (31), that the control system (1) transforms the value ranges (23) of the derived features (16) onto the intensity range (31), that

the control system (1) comprises a display (3), and, that the control system (1) displays on the display (3) the at least, preferably exactly, four dimensions of the feature space (25), and/or, **that** the control system (1) uses the feature space (25) to derive a prognosis about the success of the ablation therapy and/or to derive a proposal for a next step in the ablation therapy and/or to derive a prognosis of the change of the feature space (25) by a possible ablation step, and, that the control system (1) transforms the value ranges (23) of the derived features (16) onto the intensity range (31) via a different transformation function per feature.

2. Method according to claim 1, **characterized in that** the intervals (19) are at least 0.5 seconds long, preferably at least 1 second long, more preferably at least two seconds long, that the control system (1) displays at least 5 minutes, preferably at least 8 minutes, more preferably at least 12 minutes, of the time dimension (21), more preferably, that the control system (1) displays on the display (3) several rows of data along a common time axis (33), that the rows of data comprise a row for each of the derived features (16), that the rows comprise the data points (20) of the respective feature along the time axis (33) and mapped intensity values of the intensity range (31) for the data points (20), preferably, that the intensity values are color coded, in particular as a heatmap.

3. Method according to claim 2, **characterized in that** the control system (1) low pass filters the intensity values of the derived features (16) along the time dimension (21) and displays the low passed derived features (16), and/or, that the control system (1) visually groups the derived features (16) along the feature dimension (27) and displays the anatomical dimension (26) of each feature as a visual group (30) along the anatomical dimension (26).

4. Method according to claim 2 or 3, **characterized in that** the control system (1) further displays on the display (3) at least one channel (8) of a surface ECG (24) and/or at least one channel (8) of the intracardiac electrogram (2), preferably, that the control system (1) displays on the display (3) a section of the at least one channel (8) with a displayed time length (32) shorter than the displayed time length (32) of the time dimension (21) of the feature space (25) and/or with data points (20), in particular ECG data points (34), spaced apart less than the length of the intervals (19), more preferably, that the displayed time length (32) of the channel (8) is less than 1 minute, preferably less than 30 seconds, and/or, that the data points (20) are spaced apart less than 100 ms, preferably less than 10 ms.

5. Method according to one of the preceding claims, **characterized in that** the data points (20) of the derived features (16) are calculated over time spans (35) greater than the intervals (19), preferably, that the time spans (35) are at least ten seconds, preferably at least 20 seconds, and/or less than one minute, preferably less than 45 seconds.

6. Method according to one of the preceding claims, **characterized in that** the control system (1) further derives at least one, preferably at least two, surface features (29) from the surface ECG (24), that the surface features (29) have a time dimension (21) and a value dimension (22), that the control system (1) inserts the surface features (29) into the feature space (25) such that the feature dimension (27) comprises the surface features (29), that the anatomical dimension (26) comprises a global anatomical region, that the time dimension (21) of the surface features (29) is mapped onto the time dimension (21) of the feature space (25), that the control system (1) transforms the value ranges (23) of the value dimension (22) of the surface features (29) onto the intensity range (31).

7. Method according to one of the preceding claims, **characterized in that** the control system (1) constantly receives new data from a measurement system comprising at least the catheter (4) and updates the feature space (25), preferably, that the control system (1) updates the display (3) periodically, in particular in real time.

8. Method according to one of the preceding claims, **characterized in that** the transformation function is equal for all anatomical regions per feature, and/or, that at least one, preferably all, transformation functions are non-linear, and/or, that the transformation functions are updated based on the value ranges (23) of the respective feature or of all anatomical regions per feature over the time dimension (21) or part of the time dimension (21), in particular the displayed time dimension (21), preferably, that the part of the time dimension (21) changes with new intervals (19), in particular with every new interval (19).

9. Method according to claim 8, **characterized in that** the control system (1) adapts at least one of the transformation functions, preferably all the transformation functions, based on the value range (23) of the respective feature or feature group, in particular over the whole time dimension (21) or part of the time dimension (21), in particular the displayed part of the time dimension (21), and based on predefined boundaries, in particular boundaries for the center of the transformation function, and/or, that the control system (1) adapts at least one of the transformation functions, preferably all the transformation functions, such the transformation of the values of some data points (20) of the respective feature depends on the values of data points (20) located later in time.

10. Method according to one of the preceding claims, **characterized in that** the first group (11) and the second group (13) each comprise at least two channels (8), and/or, that the control system (1) further groups the channels (8) into at least a third group (15) of channels (8) comprising at least one, preferably at least two, channels (8) assigned to a third anatomical region (36), preferably, that the control system (1) combines at least two, preferably all, channels (8) of a, preferably every, group, in particular by calculating an average, into a single data point (20) per feature per time interval (19).

11. Method according to one of the preceding claims, **characterized in that** at least one of the features, preferably at least two, more preferably at least three, of the features of the feature dimension (27), are chosen from the group consisting of dominant frequency, autocorrelation of the surface ECG (24), a cross correlation of the intracardiac electrogram (2), cycle length, local activation time dispersion, fractionation index, cycle length variation, dominant frequency gradient.

12. Method according to one of the preceding claims, **characterized in that** the control system (1) determines the assignment of channels (8) to groups based on measured data of the intracardiac electrogram (2), in particular once per procedure, and/or, that a user may assign a subset of the channels (8) to a group.

13. Method according to one of the preceding claims, **characterized in that** the control system (1) uses the feature space (25) to derive the prognosis of the change of the feature space (25) by a possible ablation step by inputting the feature space (25) into a trained AI model.

14. Control system for analysing an intracardiac electrogram (2), configured to perform the method according to one of claims 1 to 13,

wherein the intracardiac electrogram (2) has been recorded via a catheter (4) inserted into a human body and has a time length, wherein the catheter (4) comprises multiple electrodes (7) placed at different anatomical positions, wherein multiple channels (8) of the intracardiac electrogram (2) have been recorded by the electrodes (7), wherein the control system (1) groups the channels (8) into at least a first group (11) of channels (8) comprising at least one channel (8)

assigned to a first anatomical region (12) and a second group (13) of channels (8) comprising at least one channel (8) assigned to a second anatomical region (14),

wherein the control system (1) derives derived features (16) comprising a first derived feature (17) relating to the first anatomical region (12) and a different second derived feature (18) relating to the first anatomical region (12) from the first group (11) of channels (8) and derives the first derived feature (17) and the second derived feature (18) relating to a second anatomical region (14) from the second group (13) of channels (8),

wherein data points (20) of the derived features (16) are derived in intervals (19) over the time length, the first and second features thereby each having a time dimension (21) and a value dimension (22) with a value range (23),

**characterized in**

**that** the control system (1) combines the derived features (16) into a feature space (25) comprising at least an anatomical dimension (26) and a feature dimension (27) and a time dimension (21) and an intensity dimension (28), that the anatomical dimension (26) comprises the anatomical regions, that the feature dimension (27) comprises the derived features (16), that the time dimension (21) is the time dimension (21) of the first and the second derived features (16), that the intensity dimension (28) comprises an intensity range (31), that the control system (1) transforms the value ranges (23) of the derived features (16) onto the intensity range (31),

**that** the control system (1) comprises a display (3), and, that the control system (1) displays on the display (3) the at least, preferably exactly, four dimensions of the feature space (25), and/or,

**that** the control system (1) uses the feature space (25) to derive a prognosis about the success of the ablation therapy and/or to derive a proposal for a next step in the ablation therapy and/or to derive a prognosis of the change of the feature space (25) by a possible ablation step, and, that the control system (1) transforms the value ranges (23) of the derived features (16) onto the intensity range (31) via a different transformation function per feature.

15. Control system according to claim 14, **characterized in that** the control system (1) comprises an interface to a measurement system and/or to the catheter (4) and is configured to be used during ablation therapy, in particular stepwise ablation therapy, to aid in determining a prognosis about the success of the ablation therapy.

**Patentansprüche**

1. Verfahren zum Analysieren eines intrakardialen Elektrogramms (2) über ein Steuersystem (1), wobei das intrakardiale Elektrogramm (2), insbesondere während einer Ablationstherapie, über einen in einen menschlichen Körper eingeführten Katheter (4) aufgezeichnet worden ist und eine Zeitlänge aufweist, wobei der Katheter (4) mehrere Elektroden (7) umfasst, die an unterschiedlichen anatomischen Positionen platziert sind, wobei mehrere Kanäle (8) des intrakardialen Elektrogramms (2) durch die Elektroden (7) aufgezeichnet worden sind, wobei das Steuersystem (1) die Kanäle (8) zu wenigstens einer ersten Gruppe (11) von Kanälen (8), umfassend wenigstens einen Kanal (8), der einer ersten anatomischen Region (12) zugewiesen ist, und einer zweiten Gruppe (13) von Kanälen (8), umfassend wenigstens einen Kanal (8), der einer zweiten anatomischen Region (14) zugewiesen ist, gruppiert,

wobei das Steuersystem (1) abgeleitete Merkmale (16), umfassend ein erstes abgeleitetes Merkmal (17), das sich auf die erste anatomische Region (12) bezieht, und ein anderes zweites abgeleitetes Merkmal (18), das sich auf die erste anatomische Region (12) bezieht, aus der ersten Gruppe (11) von Kanälen (8) ableitet und das erste abgeleitete Merkmal (17) und das zweite abgeleitete Merkmal (18) mit Bezug auf eine zweite anatomische Region (14) aus der zweiten Gruppe (13) von Kanälen (8) ableitet, wobei das Steuersystem (1) Datenpunkte (20) der abgeleiteten Merkmale (16) in Intervallen (19) über die Zeitlänge ableitet, wobei das erste und das zweite Merkmal dadurch jeweils eine Zeitdimension (21) und eine Wertedimension (22) mit einem Wertebereich (23) aufweisen,

**dadurch gekennzeichnet,**

**dass** das Steuersystem (1) die abgeleiteten Merkmale (16) zu einem Merkmalsraum (25) kombiniert, umfassend wenigstens eine anatomische Dimension (26) und eine Merkmalsdimension (27) und eine Zeitdimension (21) und eine Intensitätsdimension (28), dass die anatomische Dimension (26) die anatomischen Regionen umfasst, dass die Merkmalsdimension (27) die abgeleiteten Merkmale (16) umfasst, dass die Zeitdimension (21) die Zeitdimension (21) des ersten und des zweiten abgeleiteten Merkmals (16) ist, dass die Intensitätsdimension (28) einen Intensitätsbereich (31) umfasst, dass das Steuersystem (1) die Wertebereiche (23) der abgeleiteten Merkmale (16) auf den Intensitätsbereich (31) transformiert, dass das Steuersystem (1) eine Anzeige (3) umfasst und dass das Steuersystem (1) auf der Anzeige (3) die wenigstens, vorzugsweise genau vier Dimensionen des Merkmalsraums (25) anzeigt, und/oder,

**dass** das Steuersystem (1) den Merkmalsraum (25) verwendet, um eine Prognose zum Erfolg der Ablationstherapie abzuleiten und/oder einen Vorschlag für einen nächsten Schritt in der Ablationstherapie abzuleiten und/oder eine Prognose der Änderung des Merkmalsraums (25) durch einen möglichen Ablationsschritt abzuleiten, und dass das Steuersystem (1) die Wertebereiche (23) der abgeleiteten Merkmale (16) auf den Intensitätsbereich (31) über eine andere Transformationsfunktion pro Merkmal transformiert.

2. Verfahren gemäß Anspruch 1, **dadurch gekennzeichnet, dass** die Intervalle (19) wenigstens 0,5 Sekunden lang sind, vorzugsweise wenigstens 1 Sekunde lang, noch bevorzugter wenigstens zwei Sekunden lang, dass das Steuersystem (1) wenigstens 5 Minuten, vorzugsweise wenigstens 8 Minuten, noch bevorzugter wenigstens 12 Minuten der Zeitdimension (21) anzeigt, noch bevorzugter, dass das Steuersystem (1) auf der Anzeige (3) mehrere Reihen von Daten entlang einer gemeinsamen Zeitachse (33) anzeigt, dass die Datenreihen eine Reihe für jedes der abgeleiteten Merkmale (16) umfasst, dass die Reihen die Datenpunkte (20) des jeweiligen Merkmals entlang der Zeitachse (33) und abgebildete Intensitätswerte des Intensitätsbereichs (31) für die Datenpunkte (20) umfassen, vorzugsweise, dass die Intensitätswerte farbcodiert sind, insbesondere als eine Heatmap.

3. Verfahren gemäß Anspruch 2, **dadurch gekennzeichnet, dass** das Steuersystem (1) die Intensitätswerte der abgeleiteten Merkmale (16) entlang der Zeitdimension (21) tiefpassfiltert und die tiefpassgefilterten abgeleiteten Merkmale (16) anzeigt, und/oder, dass das Steuersystem (1) die abgeleiteten Merkmale (16) entlang der Merkmalsdimension (27) visuell gruppiert und die anatomische Dimension (26) jedes Merkmals als eine visuelle Gruppe (30) entlang der anatomischen Dimension (26) anzeigt.

4. Verfahren gemäß Anspruch 2 oder 3, **dadurch gekennzeichnet, dass** das Steuersystem (1) ferner auf der Anzeige (3) wenigstens einen Kanal (8) eines Oberflächen-EKGs (24) und/oder wenigstens einen Kanal (8) des intrakardialen Elektrogramms (2) anzeigt, vorzugsweise, dass das Steuersystem (1) auf der Anzeige (3) einen Abschnitt des wenigstens einen Kanals (8) mit einer angezeigten Zeitlänge (32) anzeigt, die kürzer ist als die angezeigte Zeitlänge (32) der Zeitdimension (21) des Merkmalsraums (25) und/oder mit Datenpunkten (20), insbesondere EKG-Datenpunkten (34), deren Abstand geringer ist als die Länge der Intervalle (19), noch bevorzugter, dass die angezeigte Zeitlänge (32) des Kanals (8) geringer ist als 1 Minute ist, vorzugsweise geringer als 30 Sekunden, und/oder, dass die Datenpunkte (20) weniger als 100 ms, vorzugsweise weniger als 10 ms beabstandet sind.

5. Verfahren gemäß einem der vorstehenden Ansprüche, **dadurch gekennzeichnet, dass** die Datenpunkte (20) der abgeleiteten Merkmale (16) über Zeitspannen (35) berechnet werden, die größer als die Intervalle (19) sind, vorzugsweise, dass die Zeitspannen (35) wenigstens zehn Sekunden betragen, vorzugsweise wenigstens 20 Sekunden und/oder weniger als eine Minute, vorzugsweise weniger als 45 Sekunden.

6. Verfahren gemäß einem der vorstehenden Ansprüche, **dadurch gekennzeichnet, dass** das Steuersystem (1) ferner wenigstens eine, vorzugsweise zwei Oberflächenmerkmale (29) aus dem Oberflächen-EKG (24) ableitet, dass die Oberflächenmerkmale (29) eine Zeitdimension (21) und eine Wertedimension (22) aufweisen, dass das Steuersystem (1) die Oberflächenmerkmale (29) in den Merkmalsraum (25) einsetzt, sodass die Merkmalsdimension (27) die Oberflächenmerkmale (29) umfasst, dass die anatomische Dimension (26) eine globale anatomische Region umfasst, dass die Zeitdimension (21) der Oberflächenmerkmale (29) auf die Zeitdimension (21) des Merkmalsraums (25) abgebildet wird, dass das Steuersystem (1) die Wertebereiche (23) der Wertedimension (22) der Oberflächenmerkmale (29) auf den Intensitätsbereich (31) transformiert.

7. Verfahren gemäß einem der vorstehenden Ansprüche, **dadurch gekennzeichnet, dass** das Steuersystem (1) konstant neue Daten von einem Messsystem empfängt, welches wenigstens den Katheter (4) umfasst, und den Merkmalsraum (25) aktualisiert, vorzugsweise, dass das Steuersystem (1) die Anzeige (3) periodisch, insbesondere in Echtzeit, aktualisiert.

8. Verfahren gemäß einem der vorstehenden Ansprüche, **dadurch gekennzeichnet, dass** die Transformationsfunktion für alle anatomischen Regionen pro Merkmal gleich ist, und/oder, dass wenigstens eine, vorzugsweise alle Transformationsfunktionen nichtlinear ist/sind, und/oder, dass die Transformationsfunktionen basierend auf den Wertebereichen (23) des jeweiligen Merkmals oder aller anatomischen Regionen pro Merkmal über die Zeitdimension (21) oder einen Teil der Zeitdimension (21), insbesondere die angezeigte Zeitdimension (21), aktualisiert werden, vorzugsweise, dass sich der Teil der Zeitdimension (21) mit neuen Intervallen (19), insbesondere mit jedem neuen Intervall (19), ändert.

9. Verfahren gemäß Anspruch 8, **dadurch gekennzeichnet, dass** das Steuersystem (1) wenigstens eine der Transformationsfunktionen, vorzugsweise alle Transformationsfunktionen, basierend auf dem Wertebereich (23) des jeweiligen Merkmals oder der jeweiligen Merkmalsgruppe, insbesondere über die gesamte Zeitdimension (21) oder einen Teil der Zeitdimension (21), insbesondere den angezeigten Teil der Zeitdimension (21), und basierend auf vordefinierten Grenzen, insbesondere Grenzen für die Mitte der Transformationsfunktion, anpasst, und/oder, dass das Steuersystem (1) wenigstens eine der Transformationsfunktionen, vorzugsweise alle Transformationsfunktionen, anpasst, sodass die Transformation der Werte einiger Datenpunkte (20) des jeweiligen Merkmals von den Werten von Datenpunkten (20) abhängt, die sich an einem späteren Zeitpunkt befinden.

10. Verfahren gemäß einem der vorstehenden Ansprüche, **dadurch gekennzeichnet, dass** die erste Gruppe (11) und die zweite Gruppe (13) jeweils wenigstens zwei Kanäle (8) umfassen, und/oder, dass das Steuersystem (1) ferner die Kanäle (8) zu wenigstens einer dritten Gruppe (15) von Kanälen (8) gruppiert, umfassend wenigstens einen, vorzugsweise wenigstens zwei Kanäle (8), die einer dritten anatomischen Region (36) zugewiesen sind, vorzugsweise dass das Steuersystem (1) wenigstens zwei, vorzugsweise alle Kanäle (8) einer, vorzugsweise jeder Gruppe, insbesondere durch Berechnen eines Durchschnitts, zu einem einzelnen Datenpunkt (20) pro Merkmal pro Zeitintervall (19) kombiniert.

11. Verfahren gemäß einem der vorstehenden Ansprüche, **dadurch gekennzeichnet, dass** wenigstens eines der Merkmale, vorzugsweise wenigstens zwei, noch bevorzugter wenigstens drei der Merkmale der Merkmalsdimension (27), aus der Gruppe bestehend aus dominante Frequenz, Autokorrelation des Oberflächen-EKGs (24), Kreuzkorrelation des intrakardialen Elektrogramms (2), Zykluslänge, lokale Aktivierungszeitdispersion, Fraktionierungsindex, Zykluslängenvariation, dominanter Frequenzgradient auswählt wird/werden.

12. Verfahren gemäß einem der vorstehenden Ansprüche, **dadurch gekennzeichnet, dass** das Steuersystem (1) die Zuweisung von Kanälen (8) zu Gruppen basierend auf gemessenen Daten des intrakardialen Elektrogramms (2), insbesondere einmalig pro Prozedur, bestimmt, und/oder, dass ein Benutzer eine Teilmenge der Kanäle (8) einer Gruppe zuweisen kann.

13. Verfahren gemäß einem der vorstehenden Ansprüche, **dadurch gekennzeichnet, dass** das Steuersystem (1) den Merkmalsraum (25) verwendet, um die Prognose der Änderung des Merkmalsraums (25) durch einen möglichen Ablationsschritt abzuleiten, indem der Merkmalsraum (25) in ein trainiertes KI-Modell eingegeben wird.

14. Steuersystem zum Analysieren eines intrakardialen Elektrogramms (2), das dazu ausgelegt ist, das Verfahren gemäß einem der Ansprüche 1 bis 13 durchzuführen,

wobei das intrakardiale Elektrogramm (2) über einen in einen menschlichen Körper eingeführten Katheter (4) aufgezeichnet worden ist und eine Zeitlänge aufweist, wobei der Katheter (4) mehrere Elektroden (7) umfasst, die an unterschiedlichen anatomischen Positionen platziert sind, wobei mehrere Kanäle (8) des intrakardialen Elektrogramms (2) durch die Elektroden (7) aufgezeichnet worden sind, wobei das Steuersystem (1) die Kanäle (8) zu wenigstens einer ersten Gruppe (11) von Kanälen (8), umfassend wenigstens einen Kanal (8), der einer ersten anatomischen Region (12) zugewiesen ist, und einer zweiten Gruppe (13) von Kanälen (8), umfassend wenigstens einen Kanal (8), der einer zweiten anatomischen Region (14) zugewiesen ist, gruppiert, wobei das Steuersystem (1) abgeleitete Merkmale (16), umfassend ein erstes abgeleitetes Merkmal (17), das sich auf die erste anatomische Region (12) bezieht, und ein anderes zweites abgeleitetes Merkmal (18), das sich auf die erste anatomische Region (12) bezieht, aus der ersten Gruppe (11) von Kanälen (8) ableitet und das erste abgeleitete Merkmal (17) und das zweite abgeleitete Merkmal (18) mit Bezug auf eine zweite anatomische Region (14) aus der zweiten Gruppe (13) von Kanälen (8) ableitet, wobei die Datenpunkte (20) der abgeleiteten Merkmale (16) in Intervallen (19) über eine Zeitlänge abgeleitet werden, wobei das erste und das zweite Merkmal dadurch jeweils eine Zeitdimension (21) und eine Wertedimension (22) mit einem Wertebereich (23) aufweisen, **dadurch gekennzeichnet, dass** das Steuersystem (1) die abgeleiteten Merkmale (16) zu einem Merkmalsraum (25) kombiniert, umfassend wenigstens eine anatomische Dimension (26) und eine Merkmalsdimension (27) und eine Zeitdimension (21) und eine Intensitätsdimension (28), dass die anatomische Dimension (26) die anatomischen Regionen umfasst, dass die Merkmalsdimension (27) die abgeleiteten Merkmale (16) umfasst, dass die Zeitdimension (21) die Zeitdimension (21) des ersten und des zweiten abgeleiteten Merkmals (16) ist, dass die Intensitätsdimension (28) einen Intensitätsbereich (31) umfasst, dass das Steuersystem (1) die Wertebereiche (23) der abgeleiteten Merkmale (16) auf den Intensitätsbereich (31) transformiert,

**dass** das Steuersystem (1) eine Anzeige (3) umfasst und dass das Steuersystem (1) auf der Anzeige (3) die wenigstens, vorzugsweise genau vier Dimensionen des Merkmalsraums (25) anzeigt, und/oder,

**dass** das Steuersystem (1) den Merkmalsraum (25) verwendet, um eine Prognose zum Erfolg der Ablationstherapie abzuleiten und/oder einen Vorschlag für einen nächsten Schritt in der Ablationstherapie abzuleiten und/oder eine Prognose der Änderung des Merkmalsraums (25) durch einen möglichen Ablationsschritt abzuleiten, und dass das Steuersystem (1) die Wertebereiche (23) der abgeleiteten Merkmale (16) auf den Intensitätsbereich (31) über eine andere Transformationsfunktion pro Merkmal transformiert.

15. Steuersystem gemäß Anspruch 14, **dadurch gekennzeichnet, dass** das Steuersystem (1) eine Schnittstelle zu einem Messsystem und/oder zu dem Katheter (4) umfasst und dazu ausgelegt ist, während einer Ablationstherapie, insbesondere einer schrittweisen Ablationstherapie, verwendet zu werden, um das Bestimmen einer Prognose zum Erfolg der Ablationstherapie zu unterstützen.

**Revendications**

1. Procédé d'analyse d'un électrogramme intracardiaque (2) par le biais d'un système de commande (1), dans lequel l'électrogramme intracardiaque (2) a été enregistré, en particulier lors d'une thérapie d'ablation, par le biais d'un cathéter (4) inséré dans un corps humain et présente une durée, dans lequel le cathéter (4) comprend de multiples électrodes (7) placées à différentes positions anatomiques, dans lequel de multiples canaux (8) de l'électrogramme intracardiaque (2) ont été enregistrés par les électrodes (7), dans lequel le système de commande (1) regroupe les canaux (8) en au moins un premier groupe (11) de canaux (8) comprenant au moins un canal (8) affecté à une première région anatomique (12) et un deuxième groupe (13) de canaux (8) comprenant au moins un canal (8) affecté à une deuxième région anatomique (14),

dans lequel le système de commande (1) dérive des caractéristiques dérivées (16) comprenant une première caractéristique dérivée (17) relative à la première région anatomique (12) et une seconde caractéristique dérivée (18) différente relative à la première région anatomique (12) du premier groupe (11) de canaux (8) et dérive la première caractéristique dérivée (17) et la seconde caractéristique dérivée (18) relatives à une deuxième région anatomique (14) du deuxième groupe (13) de canaux (8),

dans lequel le système de commande (1) dérive des points de données (20) des caractéristiques dérivées (16) à intervalles (19) sur la durée, les première et seconde caractéristiques ayant ainsi chacune une dimension de temps (21) et une dimension de valeur (22) avec une plage de valeurs (23),

**caractérisé en ce**

**que** le système de commande (1) combine les caractéristiques dérivées (16) dans un espace de caractéristiques (25) comprenant au moins une dimension anatomique (26) et une dimension de caractéristiques (27) et une dimension de temps (21) et une dimension d'intensité (28), que la dimension anatomique (26) comprend les régions anatomiques, que la dimension de caractéristiques (27) comprend les caractéristiques dérivées (16), que la dimension de temps (21) est la dimension de temps (21) des première et seconde caractéristiques dérivées (16), que la dimension d'intensité (28) comprend une plage d'intensité (31), que le système de commande (1) transforme les plages de valeurs (23) des caractéristiques dérivées (16) sur la plage d'intensité (31), que le système de commande (1) comprend un écran (3), et que le système de commande (1) affiche sur l'écran (3) les quatre dimensions au moins, de préférence exactement, de l'espace de caractéristiques (25), et/ou,

**que** le système de commande (1) utilise l'espace de caractéristiques (25) pour dériver un pronostic sur le succès de la thérapie d'ablation et/ou pour dériver une proposition pour une étape suivante de la thérapie d'ablation et/ou pour dériver un pronostic de la modification de l'espace de caractéristiques (25) par une éventuelle étape d'ablation, et, que le système de commande (1) transforme les plages de valeurs (23) des caractéristiques dérivées (16) sur la plage d'intensité (31) par le biais d'une fonction de transformation différente pour chaque caractéristique.

2. Procédé selon la revendication 1, **caractérisé en ce que** les intervalles (19) sont longs d'au moins 0,5 seconde, de préférence longs d'au moins 1 seconde, plus préférablement longs d'au moins deux secondes, que le système de commande (1) affiche au moins 5 minutes, de préférence au moins 8 minutes, plus préférablement au moins 12 minutes, de la dimension de temps (21), plus préférablement, que le système de commande (1) affiche sur l'écran (3) plusieurs lignes de données le long d'un axe temporel commun (33), que les lignes de données comprennent une ligne pour chacune des caractéristiques dérivées (16), que les lignes comprennent les points de données (20) de la caractéristique respective le long de l'axe de temps (33) et les valeurs d'intensité mappées de la plage d'intensité (31) pour les points de données (20), de préférence, que les valeurs d'intensité sont codées en couleur, en particulier en tant que carte thermique.

3. Procédé selon la revendication 2, **caractérisé en ce que** le système de commande (1) applique un filtre passe-bas aux valeurs d'intensité des caractéristiques dérivées (16) au cours de la dimension de temps (21) et affiche les caractéristiques dérivées (16) auxquelles a été appliqué le filtre passe-bas, et/ou, que le système de commande (1) regroupe visuellement les caractéristiques dérivées (16) le long de la dimension de caractéristiques (27) et affiche la dimension anatomique (26) de chaque caractéristique en tant que groupe visuel (30) le long de la dimension anatomique (26).

4. Procédé selon la revendication 2 ou 3, **caractérisé en ce que** le système de commande (1) affiche en outre sur l'écran (3) au moins un canal (8) d'un ECG de surface (24) et/ou au moins un canal (8) de l'électrogramme intracardiaque (2), de préférence, que le système de commande (1) affiche sur l'écran (3) une section de l'au moins un canal (8) avec une durée affichée (32) plus courte que la durée affichée (32) de la dimension de temps (21) de l'espace de caractéristiques (25) et/ou avec des points de données (20), en particulier des points de données ECG (34), espacés de moins de la longueur des intervalles (19), plus préférablement, que la durée affichée (32) du canal (8) est inférieure à 1 minute, de préférence inférieure à 30 secondes, et/ou, que les points de données (20) sont espacés de moins de 100 ms, de préférence de moins de 10 ms.

5. Procédé selon l'une des revendications précédentes, **caractérisé en ce que** les points de données (20) des caractéristiques dérivées (16) sont calculés sur des périodes de temps (35) supérieures aux intervalles (19), de préférence, que les périodes de temps (35) sont d'au moins dix secondes, de préférence d'au moins 20 secondes, et/ou inférieures à une minute, de préférence inférieures à 45 secondes.

6. Procédé selon l'une des revendications précédentes, **caractérisé en ce que** le système de commande (1) dérive en outre au moins une, de préférence au moins deux, caractéristiques de surface (29) de l'ECG de surface (24), que les caractéristiques de surface (29) ont une dimension de temps (21) et une dimension de valeur (22), que le système de commande (1) insère les caractéristiques de surface (29) dans l'espace de caractéristiques (25) de telle sorte que la dimension de caractéristiques (27) comprenne les caractéristiques de surface (29), que la dimension anatomique (26) comprend une région anatomique globale, que la dimension de temps (21) des caractéristiques de surface (29) est mappée sur la dimension de temps (21) de l'espace de caractéristiques (25), que le système de commande (1) transforme les plages de valeurs (23) de la dimension de valeur (22) des caractéristiques de surface (29) sur la plage d'intensité (31).

7. Procédé selon l'une des revendications précédentes, **caractérisé en ce que** le système de commande (1) reçoit en permanence de nouvelles données d'un système de mesure comprenant au moins le cathéter (4) et met à jour l'espace de caractéristiques (25), de préférence, que le système de commande (1) met à jour l'écran (3) périodiquement, en particulier en temps réel.

8. Procédé selon l'une des revendications précédentes, **caractérisé en ce que** la fonction de transformation est égale pour toutes les régions anatomiques par caractéristique, et/ou, qu'au moins une fonction de transformation, de préférence toutes, sont non linéaires, et/ou, que les fonctions de transformation sont mises à jour sur la base des plages de valeurs (23) de la caractéristique respective ou de toutes les régions anatomiques par caractéristique sur la dimension de temps (21) ou une partie de la dimension de temps (21), en particulier la dimension de temps (21) affichée, de préférence, que la partie de la dimension de temps (21) change avec de nouveaux intervalles (19), en particulier avec chaque nouvel intervalle (19).

9. Procédé selon la revendication 8, **caractérisé en ce que** le système de commande (1) adapte au moins une des fonctions de transformation, de préférence toutes les fonctions de transformation, sur la base de la plage de valeurs (23) de la caractéristique respective ou du groupe de caractéristiques respectif, en particulier sur l'ensemble de la dimension de temps (21) ou une partie de la dimension de temps (21), en particulier la partie affichée de la dimension de temps (21), et sur la base de limites prédéfinies, en particulier des limites pour le centre de la fonction de transformation, et/ou, que le système de commande (1) adapte au moins une des fonctions de transformation, de préférence toutes les fonctions de transformation, de telle sorte que la transformation des valeurs de certains points de données (20) de la caractéristique respective dépende des valeurs de points de données (20) situés plus tard dans le temps.

10. Procédé selon l'une des revendications précédentes, **caractérisé en ce que** le premier groupe (11) et le deuxième groupe (13) comprennent chacun au moins deux canaux (8), et/ou, que le système de commande (1) regroupe en outre les canaux (8) en au moins un troisième groupe (15) de canaux (8) comprenant au moins un canal, de préférence au moins deux canaux (8) affectés à une troisième région anatomique (36), de préférence, que le système de commande (1) combine au moins deux canaux, de préférence tous les canaux (8) d'un groupe, de préférence de chaque groupe, en particulier en calculant une moyenne, en un point de données (20) unique par caractéristique par intervalle de temps (19).

11. Procédé selon l'une des revendications précédentes, **caractérisé en ce qu'**au moins une des caractéristiques, de préférence au moins deux, plus préférablement au moins trois, des caractéristiques de la dimension de caractéristique (27), sont choisies dans le groupe consistant en fréquence dominante, autocorrélation de l'ECG de surface (24), corrélation croisée de l'électrogramme intracardiaque (2), longueur de cycle, dispersion de temps d'activation local, indice de fractionnement, variation de longueur de cycle, gradient de fréquence dominante.

12. Procédé selon l'une des revendications précédentes, **caractérisé en ce que** le système de commande (1) détermine l'affectation de canaux (8) à des groupes sur la base de données mesurées de l'électrogramme intracardiaque (2), en particulier une fois par intervention, et/ou, **en ce qu'**un utilisateur peut affecter un sous-ensemble des canaux (8) à un groupe.

13. Procédé selon l'une des revendications précédentes, **caractérisé en ce que** le système de commande (1) utilise l'espace de caractéristiques (25) pour dériver le pronostic de la modification de l'espace de caractéristiques (25) par une étape d'ablation possible en entrant l'espace de caractéristiques (25) dans un modèle d'IA entraîné.

14. Système de commande permettant d'analyser un électrogramme intracardiaque (2), configuré pour réaliser le procédé selon l'une des revendications 1 à 13,

dans lequel l'électrogramme intracardiaque (2) a été enregistré par un cathéter (4) inséré dans un corps humain et a une durée, dans lequel le cathéter (4) comprend plusieurs électrodes (7) placées à différentes positions anatomiques, dans lequel de multiples canaux (8) de l'électrogramme intracardiaque (2) ont été enregistrés par les électrodes (7), dans lequel le système de commande (1) regroupe les canaux (8) en au moins un premier groupe (11) de canaux (8) comprenant au moins un canal (8) affecté à une première région anatomique (12) et un deuxième groupe (13) de canaux (8) comprenant au moins un canal (8) affecté à une deuxième région anatomique (14),

dans lequel le système de commande (1) dérive des caractéristiques dérivées (16) comprenant une première caractéristique dérivée (17) relative à la première région anatomique (12) et une seconde caractéristique dérivée (18) différente relative à la première région anatomique (12) du premier groupe (11) de canaux (8) et dérive la première caractéristique dérivée (17) et la seconde caractéristique dérivée (18) relatives à une deuxième région anatomique (14) du deuxième groupe (13) de canaux (8),

dans lequel les points de données (20) des caractéristiques dérivées (16) sont dérivés dans des intervalles (19) sur la durée, les première et seconde caractéristiques ayant ainsi chacune une dimension de temps (21) et une dimension de valeur (22) avec une plage de valeurs (23), **caractérisé en ce**

**que** le système de commande (1) combine les caractéristiques dérivées (16) en un espace de caractéristiques (25) comprenant au moins une dimension anatomique (26) et une dimension de caractéristiques (27) et une dimension de temps (21) et une dimension d'intensité (28), que la dimension anatomique (26) comprend les régions anatomiques, que la dimension de caractéristiques (27) comprend les caractéristiques dérivées (16), que la dimension de temps (21) est la dimension de temps (21) des première et seconde caractéristiques dérivées (16), que la dimension d'intensité (28) comprend une plage d'intensité (31), que le système de commande (1) transforme les plages de valeurs (23) des caractéristiques dérivées (16) sur la plage d'intensité (31),

**que** le système de commande (1) comprend un écran (3), et, que le système de commande (1) affiche sur l'écran (3) les au moins, de préférence exactement, quatre dimensions de l'espace de caractéristiques (25), et/ou,

**que** le système de commande (1) utilise l'espace de caractéristiques (25) pour dériver un pronostic sur le succès de la thérapie d'ablation et/ou pour dériver une proposition pour une étape suivante de la thérapie d'ablation et/ou pour dériver un pronostic de la modification de l'espace de caractéristiques (25) par une éventuelle étape d'ablation, et, que le système de commande (1) transforme les plages de valeurs (23) des caractéristiques dérivées (16) sur la plage d'intensité (31) par le biais d'une fonction de transformation différente pour chaque caractéristique.

15. Système de commande selon la revendication 14, **caractérisé en ce que** le système de commande (1) comprend une interface avec un système de mesure et/ou avec le cathéter (4) et est configuré pour être utilisé pendant la thérapie d'ablation, en particulier la thérapie d'ablation par étapes, pour aider à déterminer un pronostic sur le succès de la thérapie d'ablation.

Fig. 1

Fig. 2

Fig. 3

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180368713 A1

- US 20070232949 A1